# FASCICULE DE BREVET EUROPEEN

(11) **EP 2 254 952 B1**
(45) Date de publication et mention de la délivrance du brevet: **04.07.2012**
(21) Numéro de dépôt: 09720781.5
(22) Date de dépôt: 27.02.2009
(51) Int. Cl.: C09B 61/00, C07D 311/62, A61K 9/00, A61K 47/10

(54) **COLORANT PHARMACEUTIQUE EXTRAIT DE CRANBERRY CONCENTRE EN PROANTHOCYANIDINES A**
AUS MOOSBEEREN EXTRAHIERTER PHARMAZEUTISCHER FARBSTOFF MIT HOHER PROANTHOCYANIDIN A-KONZENTRATION
PHARMACEUTICAL DYE EXTRACTED FROM CRANBERRY WITH HIGH PROANTHOCYANIDIN A CONCENTRATION

(30) Priorité: 27.02.2008 FR 0851256
(43) Date de publication de la demande: 01.12.2010
(73) Titulaire: Unither Developpement, 80080 Amiens (FR)
(72) Inventeur: MAURY, Marc, F-33160 Saint Medard En Jalles (FR); PEROVITCH, Philippe, F-33680 Le Temple (FR); GALPERINE, Tatiana, F-75009 Paris (FR)
(74) Mandataire: Cenatiempo, Julie Adeline Anne
(86) Numéro de dépôt international: PCT/FR2009/050311
(87) Numéro de publication internationale: WO 2009/112769

(56) Documents cités:
- EP-A- 1 491 191
- EP-A- 1 541 037
- WO-A-97/36497
- WO-A-99/12541
- AMY B. HOWELL, JESS D. REED, CHRISTIAN G. KRUEGER, RANEE WINTERBOTTOM, DAVID G. CUNNINGHAM, MARGE LEAHY: "A-type cranberry proanthocyanidins and uropathogenic bacterial anti-adhesion activity" PHYTOCHEMISTRY, vol. 66, no. 18, 1 août 2005 (2005-08-01), pages 2281-2291, XP002507858
- DATABASE WPI Week 199948 Thomson Scientific, London, GB; AN 1999-566575 XP002507860 & JP 11 246562 A (AGENCY OF IND SCI & TECHNOLOGY) 14 septembre 1999 (1999-09-14)
- G-I NONAKA ET AL: "Tannins and Related Compounds. L. Structures of Proanthocyanidin A-1 and Related Compounds" 1 janvier 1987 (1987-01-01), CHEMICAL AND PHARMACEUTICAL BULLETIN, PHARMACEUTICAL SOCIETY OF JAPAN, TOKYO, PAGE(S) 149 - 155 , XP008066758 ISSN: 0009-2363 page 150 page 153 page 154
- RONALD L. PRIOR, SHERYL A. LAZARUS, GUOHUA CAO, HELEN MUCCITELLI, AND JOHN F. HAMMERSTONE: "Identification of Procyanidins and Anthocyanins in Blueberries and Cranberries (Vaccinium Spp.) Using High-Performance Liquid Chromatography/Mass Spectrometry" JOURNAL OF AGRICULTURAL AND FOOD CHEMISTRY, vol. 49, no. 3, 30 janvier 2001 (2001-01-30), pages 1270-1276, XP002507859

## Description

La présente invention concerne l'utilisation de molécules particulières extraites de Cranberry comme colorant pharmaceutique, notamment l'utilisation d'une concentration de fractions spécifiques de ces molécules.

L'invention se rapporte également à un colorant pharmaceutique extrait de Cranberry comprenant ces molécules et aux produits pharmaceutiques contenant ce colorant extrait de Cranberry.

En termes de coloration des formes galéniques, l'industrie pharmaceutique est confrontée à une triple exigence : la qualité technique intrinsèque des ingrédients utilisés, avec une exigence majeure d'absence d'interactions entre excipients et/ou principes actifs ; la stabilité proprement dite du colorant ; et la non toxicité du colorant, afin de limiter l'apparition ou la résurgence de mécanismes d'intolérance locale ou systémique.

La plupart des colorants pharmaceutiques connus sont d'origine synthétique et font très souvent l'objet de recommandations officielles de précautions ou de mises en garde. En particulier pour la couleur rouge, la disponibilité et la sécurité des colorants stables est réduite. A titre d'exemple parmi les colorants rouge utilisés dans des préparations pharmaceutiques, on peut citer le rouge cochenille, acide carminique issu de l'insecte hémiptère ou synthétisé chimiquement, l'Erythrosine (interdit aux Etats-Unis et en Norvège), l'amarante (interdit aux Etats-Unis, en Autriche en Norvège et en Russie), l'orseille orcéine (interdit en France), etc.

Or, aujourd'hui, à la fois en accord avec les exigences réglementaires et les demandes de consommateurs, l'industrie pharmaceutique se trouve dans l'obligation de développer des médicaments comportant le moins possible d'excipients déclarés à effet notoire et de limiter l'utilisation de substances d'origine chimique ou animale.

Il existe peu de colorants pharmaceutiques d'origine naturelle. Parmi les colorants produits de façon naturelle, seuls les colorants de type anthocyanes présentent une couleur qui approche du rouge.

Les anthocyanes sont des colorants naturels extraits de végétaux utilisés dans l'industrie alimentaire. Il existe de nombreux dérivés des anthocyanes fournissant des teintes variées, comme la pélargonidine, la cyanidine, la péonidine, la delphinidine, la pétudine et la malvidine.

Toutefois ces colorants présentent de nombreux inconvénients et sont quasiment inemployés dans l'industrie pharmaceutique en raison de leur grande complexité ou polymorphisme chimique qui les rend difficiles à standardiser. Leur production est limitée par les prix de production des végétaux dont elles sont extraites et par leurs volumes disponibles sur les marchés en fonction des aléas saisonniers, mais également par des difficultés et coûts d'extraction, de conservation et/ou de stabilisation. En outre, leurs structures polymériques très polymorphes et de fort poids moléculaire restent sensibles aux effets des rayonnements ultraviolets ainsi qu'aux phénomènes d'oxydation. En particulier, selon le pH auquel elles se trouvent soumises, les Anthocyanes peuvent présenter une variation significative de leur coloration exprimée, laquelle fluctue du rouge en pH relativement acide ou neutre pour tourner vers le bleu en pH basique et vers le jaune en pH très basique, ce en raison de modifications des structures électroniques périphériques de leurs cycles polymériques.

Aussi, la présente invention vise à pallier aux inconvénients de l'art antérieur en proposant un colorant rouge naturel répondant à toutes les exigences de qualité pharmaceutique. L'objectif est de proposer un colorant pharmaceutique naturel sans risques d'intolérances et présentant des qualités colorantes stables compatibles avec la plupart des excipients usuels des formulations pharmaceutiques liquides ou solides.

A cet effet, l'invention a pour objet l'utilisation d'extraits concentrés en fractions spécifiques de Proanthocyanidines A extraites de Cranberry pour la coloration de produits pharmaceutiques.

Les Proanthocyanidines de type A présentent une teinte rouge violet intense. Les Proanthocyanidines sont connues pour certains usages pharmaceutiques.

La demande WO-99/12541 décrit notamment l'utilisation de Proanthocyanidines pour empêcher l'adhésion de bactéries sur des membranes et lutter contre des infections urinaires.

La demande EP-1.491.191 décrit également un extrait de litchi contenant des Proanthocyanidines oligomériques et son utilisation pour la fabrication de préparations pharmaceutiques.

Les Proanthocyanidines A sont présents en forte concentration dans les baies de Cranberry (fruits de canneberge).

Toutefois les extraits de Cranberry actuellement connus sont faiblement dosés en Proanthocyanidines et contiennent d'importantes quantités d'acides organiques et de sucres qui les rendent difficilement utilisables en tant que colorant.

En particulier, l'utilisation de ces extraits connus de Cranberry dans un sirop pharmaceutique pour obtenir une coloration rouge confère à la préparation un pH très acide. Ce pH très acide rend difficile la réalisation des formulations pharmaceutiques et les rend instables. L'efficacité des conservateurs comme ceux de la famille des parabens est affaiblie pour des pH inférieur à 5 ou supérieur à 7. De la même manière les pH bas rendent difficile l'optimisation des aromatisations puisque seuls les colorants issus d'agrumes sont compatibles avec des pH très bas. Enfin la stabilité de nombreux principes actifs peut également se trouver affectée par un pH acide qui serait imposé lors de l'utilisation d'un excipient.

C'est pourquoi la présente invention propose d'utiliser comme agent colorant pharmaceutique un extrait spécifique de Cranberry hautement purifié, un concentré comprenant au moins 35% de Proanthocyanidines A.

En particulier l'invention vise l'utilisation comme agent colorant pharmaceutique d'un extrait de Cranberry comprenant au moins 35% de Proanthocyanidines A, concentré obtenu à l'issue d'opérations successives renouvelées de purifications sélectives et majoritairement constitué de polymères de poids moléculaire inférieur à 6000 daltons, préférentiellement de poids moléculaire compris entre 400 et 1200 daltons.

Le pourcentage de Proanthocyanidines A pour la présente invention correspond au pourcentage de Proanthocyanidines A en poids de l'extrait sec, c'est-à-dire au pourcentage de Proanthocyanidines A en poids d'agent colorant.

L'invention vise également le colorant spécifique issu de Cranberry ainsi que les préparations pharmaceutiques colorées l'incluant.

Avantageusement la présente invention permet la mise en oeuvre de préparations pharmaceutiques colorimétriquement stables, que ce soit en phases liquides , visqueuses ou encore solides ou pulvérulentes. De par sa composition très particulière, l'extrait de Cranberry concentré en Proanthocyanidines A, notamment en polymères de faible poids moléculaire, présente l'avantage technique de conserver sa coloration rouge stable même lorsqu'il se trouve exposé à des variations significatives de pH, par exemple entre 3 et 10. Il est donc possible de l'utiliser sans aléas dans des procédés de préparations industrielles de certaines compositions qui imposent des phases successives de préparations très acides et/ou très basiques, ce qui n'est actuellement pas possible avec les colorants utilisés en pharmacie.

De plus, le colorant selon l'invention, d'origine naturelle, est dépourvu d'effets nocifs pour la santé publique, les jus et préparations alimentaires à base de Cranberry étant reconnus depuis plusieurs siècles pour leurs bienfaits alimentaires et préventifs d'affections, allégations reconnues par les autorités sanitaires.

D'autres caractéristiques et avantages ressortiront de la description en détail qui va suivre.

Selon l'invention, les Proanthocyanidines A issues de Cranberry sont utilisées pour la coloration de préparations pharmaceutiques.

En particulier l'invention vise l'utilisation d'un extrait de Cranberry concentré en Proanthocyanidines A en tant qu'agent colorant dans des préparations pharmaceutiques.

Le colorant pharmaceutique selon l'invention est un extrait de Cranberry concentré en Proanthocyanidines A, comprenant au moins 35% de Proanthocyanidines A.

Préférentiellement l'extrait spécifique de Cranberry est concentré en certains polymères de Proanthocyanidines A qui le constituent dans une proportion d'au moins 35%. Les molécules de Proanthocyanidines A sont majoritairement des polymères de faible poids moléculaire. Par polymères de faible poids moléculaire on entend dimères, trimères, tétramères, pentamères, hexamères, heptamères, octamères, nonamères et/ou décamères et/ou tout polymère en dessous de 6 000 daltons. Au-delà, les polymères de grosses dimensions peuvent générer des aléas agrégatifs ou des déséquilibres structurels des mélanges. De façon préférée il s'agit de dimères, trimères, tétramères, pentamères, hexamères, heptamères et/ou octamères avec un poids moléculaire compris entre 400 et 1200 Daltons.

Selon un mode de réalisation, les baies de canneberge utilisées sont de la variété Vaccinium Macrocarpon, Cranberry nord américain.

L'extrait de Cranberry purifié et concentré en Proanthocyanidines A, selon l'invention peut être obtenu par un procédé comportant la succession d'étapes suivantes :
- adsorption des jus de Cranberry sur résines,
- élution à l'alcool, et
- concentration puis extraction hydro-alcoolique reconduite à partir de ces mêmes concentrés secs.

Des exemples de mode d'extraction sont connus et décrits notamment dans la demande de brevet européen EP 1 913 951. Ces modes sont donc intégrés à la présente demande de brevet à titre d'exemples de successions d'étapes permettant à l' homme de l'art d'obtenir des extraits de Cranberry purifiés et concentrés en Proanthocyanidines A.

Avantageusement, le colorant pharmaceutique selon l'invention répond à toutes les exigences de qualité pharmaceutique.

Comme il s'agit d'un extrait de Cranberry, il existe une grande sécurité en termes de volumes et de qualité de production agricole, avec une qualité protégée puisque dépourvue de pesticides, pollutions environnementales et/ou résidus d'additifs agro-chimiques.

De plus cette substance d'origine végétale présente une absence totale de toxicité organique.

En outre le colorant selon l'invention permet d'obtenir une coloration rouge optimale stabilisée dans un large gradient de pH, entre 3 et 10.

Selon un dernier aspect, l'invention vise également les préparations pharmaceutiques colorées en rouge de manière stable avec un concentré, extrait de Cranberry comprenant au moins 35% de Proanthocyanidines A en poids. Les polymères de Proanthocyanidines A du concentré selon l'invention génèrent la coloration rouge.

Préférentiellement l'invention vise les préparations pharmaceutiques colorées en rouge par un colorant, extrait spécifique de Cranberry concentré en polymères de Proanthocyanidines A de poids moléculaire inférieur à 6000 daltons, qui le constituent dans une proportion d'au moins 35% en poids d'agent colorant. Encore plus préférentiellement il s'agit de préparations pharmaceutiques colorées en rouge par un colorant extrait spécifique de Cranberry concentré en polymères (dimères, trimères, tétramères, pentamères, hexamères, heptamères et/ou octamères) de Proanthocyanidines A de poids moléculaire compris entre 400 et 1200 Daltons, qui le constituent dans une proportion d'au moins 35% en poids.

Par préparations pharmaceutiques on entend toutes compositions thérapeutiques, solides, pulvérulentes, liquides ou visqueuses. Ainsi on peut citer notamment des comprimés à sucer ou à ingérer, des formes obtenues par lyophilisation à délitement buccal, des dragées, des comprimés effervescents, des solutions ou suspensions buvables, des crèmes ou gels pour applications cutanées et muqueuses, des solutions hydro-alcooliques, des matériaux colloïdes ou des formulations composites pour les soins des plaies et pansements. Préférentiellement, ces préparations pharmaceutiques sont des formes sirops ou solutés buvables médicamenteux.

Les exemples qui suivent montrent différentes formulations possibles de sirops ou de solutés buvables médicamenteux comprenant le colorant pharmaceutique riche en Proanthocyanidines A selon l'invention.

### Exemple 1

| | |
|---|---|
| Principe actif | QS |
| Saccharose | 60 g |
| Parahydroxybenzoate de méthyle sodé | 0,100 g |
| Parahydroxybenzoate de propyle sodé | 0,05 g |
| Arome fraise | 0,100 mg |
| Colorant selon l'invention | 0,015 g |
| Eau purifiée | QS 100 ml |

### Exemple 2

| | |
|---|---|
| Principe actif 1 | QS |
| Principe actif 2 | QS |
| Saccharinate de sodium | 0,015 g |
| Parahydroxybenzoate de méthyle sodé | 0,150 g |
| Arome fruits rouges | 0,150 mg |
| Colorant selon l'invention | 0,015 g |
| Eau purifiée | QS 100 ml |

### Exemple 3

| | |
|---|---|
| Principe actif 1 | QS |
| Principe actif 2 | QS |
| Saccharinate de sodium | 0,015 g |
| Hydroxyméthyle cellulose sodique | 0,030 g |
| Parahydroxybenzoate de méthyle sodé | 0,150 g |
| Arome fruits rouges | 0,150 mg |
| Colorant selon l'invention | 0,015 g |
| Eau purifiée | QS 100 ml |

### Exemple 4

| | |
|---|---|
| Principe actif 1 | QS |
| Saccharinate de sodium | 0,015 g |
| Hydroxyméthyle cellulose sodique | 0,030 g |
| Parahydroxybenzoate de méthyle sodé | 0,150 g |
| Arome fruits rouges | 0,150 mg |
| Colorant selon l'invention | 0,015 g |
| Eau purifiée | QS 100 ml |

Le procédé de fabrication de ces préparations pharmaceutiques consiste en une simple dissolution des différents constituants.

A titre d'illustration, pour l'exemple 1, le procédé de fabrication consiste en la succession des étapes suivantes :
- Dans une cuve en acier inoxydable introduire l'eau purifiée,
- Introduire sous agitation le saccharose et agiter la préparation jusqu'à dissolution complète,
- Introduire les conservateurs parabens sodés et agiter jusqu'à dissolution complète,
- Introduire le colorant selon l'invention extrait de Cranberry comprenant au moins 35% de Proanthocyanidines A,
- Mélanger la préparation jusqu'à l'obtention d'une coloration homogène, et
- Filtrer la préparation sur un filtre clarifiant de 50 µm de porosité.

Ainsi les Proanthocyanidines A selon l' invention sont introduites préférentiellement entre 35 et 99%.

## Revendications

1. Utilisation d'un extrait de Cranberry comprenant au moins 35% de Proanthocyanidines A pour la coloration de préparations pharmaceutiques.

2. Utilisation selon la revendication 1, **caractérisée en ce que** les molécules de Proanthocyanidines A sont majoritairement des polymères de poids moléculaire inférieur à 6000 daltons.

3. Utilisation selon la revendication 1, **caractérisée en ce que** les molécules de Proanthocyanidines A sont majoritairement des polymères de poids moléculaire compris entre 400 et 1200 daltons.

4. Utilisation selon la revendication 3, **caractérisée en ce que** les polymères sont majoritairement des dimères, trimères, tétramères, pentamères, hexamères, heptamères et/ou octamères.

5. Colorant pharmaceutique naturel, **caractérisé en ce qu'**il s'agit d'un extrait de Cranberry comprenant au moins 35% de Proanthocyanidines A en poids.

6. Colorant pharmaceutique selon la revendication 5, **caractérisé en ce que** les molécules de Proanthocyanidines A dudit extrait sont majoritairement des polymères de poids moléculaire inférieure à 6000 daltons.

7. Colorant pharmaceutique selon la revendication 5 ou 6, **caractérisé en ce que** les molécules de Proanthocyanidines A dudit extrait sont majoritairement des polymères de poids moléculaire compris entre 400 et 1200 daltons.

8. Colorant pharmaceutique selon l'une des revendications 5 à 7, **caractérisé en ce qu'**il s'agit d'un extrait de Vaccinium Macrocarpon comprenant au moins 35% de Proanthocyanidines A en poids.

9. Préparation pharmaceutique colorée en rouge comprenant un colorant selon l'une des revendications 5 à 8.

10. Préparation pharmaceutique colorée en rouge selon la revendication 9, **caractérisée en ce qu'**elle se présente sous forme de sirop ou soluté buvable médicamenteux.

## Claims

1. Use of a cranberry extract comprising at least 35% A-type proanthocyanidins for colouring pharmaceutical preparations.

2. Use according to claim 1, **characterised in that** the A-type proanthocyanidin molecules are mainly polymers with a molecular weight of less than 6000 daltons.

3. Use according to claim 1, **characterised in that** the A-type proanthocyanidin molecules are mainly polymers with a molecular weight of between 400 and 1200 daltons.

4. Use according to claim 3, **characterised in that** the polymers are mainly dimers, trimers, tetramers, pentamers, hexamers, heptamers and/or octamers.

5. A natural pharmaceutical colouring, **characterised in that** it is an extract of cranberry comprising at least 35% A-type proanthocyanidins by weight.

6. A pharmaceutical colouring according to claim 5, **characterised in that** the A-type proanthocyanidin molecules of said extract are mainly polymers with a molecular weight of less than 6000 daltons.

7. A pharmaceutical colouring according to claim 5 or 6, **characterised in that** the A-type proanthocyanidin molecules of said extract are mainly polymers with a molecular weight of between 400 and 1200 daltons.

8. A pharmaceutical colouring according to one of claims 5 to 7, **characterised in that** it is an extract of Vaccinium macrocarpon comprising at least 35% A-type proanthocyanidins by weight.

9. A red-coloured pharmaceutical preparation comprising a colouring according to one of claims 5 to 8.

10. A red-coloured pharmaceutical preparation according to claim 9, **characterised in that** it is in the form of a medicinal potable syrup or solute.

## Patentansprüche

1. Verwendung eines Moosbeerenextrakts mit wenigstens 35 % Proanthocyanidin A zum Färben von pharmazeutischen Präperaten.

2. Verwendung nach Anspruch 1, **dadurch gekennzeichnet, dass** die Moleküle von Proanthocyanidin A mehrheitlich Polymere mit einem Molekulargewicht unterhalb von 6000 Dalton sind.

3. Verwendung nach Anspruch 1, **dadurch gekennzeichnet, dass** die Moluküle von Proanthocyanidin A mehrheitlich Polymere mit einem Molekulargewicht zwischen 400 und 1200 Dalton sind.

4. Verwendung nach Anspruch 3, **dadurch gekennzeichnet, dass** die Polymere mehrheitlich Dimere, Trimere, Tetramere, Pentamere, Hexamere, Heptamere und/oder Oktamere sind.

5. Natürlicher pharmazeutischer Farbstoff, **dadurch gekennzeichnet, dass** es sich um ein Moosbeerenextrakt mit wenigstens 35 Gew.-% an Proanthocyanidin A handelt.

6. Pharmazeutischer Farbstoff nach Anspruch 5, **dadurch gekennzeichnet, dass** die Moleküle von Proanthocyanidin A des Extrakts mehrheitlich Polymere mit einem Molekülgewicht unterhalb von 6000 Dalton sind.

7. Pharmazeutischer Farbstoff nach Anspruch 5 oder 6, **dadurch gekennzeichnet, dass** die Moleküle von Proanthocyanidin A des Extrakts mehrheitlich Polymere mit einem Molekülgewicht zwischen 400 und 1200 Dalton sind.

8. Pharmazeutischer Farbstoff nach einem der Ansprüche 5 bis 7, **dadurch gekennzeichnet, dass** es sich um ein Extrakt von Vaccinium macrocarpon mit wenigstens 35 Gew.-% an Proanthocyanidin A handelt.

9. Rotgefärbtes pharmazeutisches Präparat, das einen Farbstoff nach einem der Ansprüche 5 bis 8 aufweist.

10. Rotgefärbtes pharmazeutisches Präparat nach Anspruch 9, **dadurch gekennzeichnet, dass** es in Form eines Sirups oder eines medikamentösen trinkbaren gelösten Stoffs vorlegt.
